(19) Europäisches Patentamt
European Patent Office
Office européen des brevets

(11) **EP 2 810 651 B1**

(12) **EUROPEAN PATENT SPECIFICATION**

(45) Date of publication and mention of the grant of the patent:
**11.09.2019 Bulletin 2019/37**

(21) Application number: **14170846.1**

(22) Date of filing: **02.06.2014**

(51) Int Cl.:
*A61K 36/88* (2006.01)   *A61K 36/28* (2006.01)
*A61P 1/16* (2006.01)   *A61P 17/00* (2006.01)
*A61P 13/12* (2006.01)   *A61P 31/00* (2006.01)
*A61K 8/60* (2006.01)   *A61Q 19/02* (2006.01)
*A61K 8/97* (2017.01)   *A61K 31/7032* (2006.01)

(54) **Galactolipids-enriched plant extracts and the uses thereof**

Mit Galactolipiden angereicherte Pflanzenextrakte und Verwendungen davon

Extraits végétaux enrichis aux galactolipides et leurs utilisations

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.06.2013 TW 102119755**

(43) Date of publication of application:
**10.12.2014 Bulletin 2014/50**

(73) Proprietor: **Academia Sinica**
**Nankang, Taipei 115 (TW)**

(72) Inventors:
• **Shyur, Lie-Fen**
**115 Taipei (TW)**
• **Feng, Jia-Hua**
**115 Taipei (TW)**
• **Apaya, Maria Karmella**
**221 New Taipei City (TW)**

(74) Representative: **Lang, Christian**
**LangPatent Anwaltskanzlei**
**Ingolstädter Straße 5**
**80807 München (DE)**

(56) References cited:
**WO-A1-2008/105436    WO-A1-2012/021068**
**US-A1- 2008 152 737**

• **C.-C. HOU ET AL: "A Galactolipid Possesses Novel Cancer Chemopreventive Effects by Suppressing Inflammatory Mediators and Mouse B16 Melanoma", CANCER RESEARCH, vol. 67, no. 14, 15 July 2007 (2007-07-15) , pages 6907-6915, XP055135984, ISSN: 0008-5472, DOI: 10.1158/0008-5472.CAN-07-0158**

• **PHAN VAN KIEM ET AL: "Inhibitory effect on TNF-[alpha]-induced IL-8 secretion in HT-29 cell line by glyceroglycolipids from the leave", ARCHIVES OF PHARMACAL RESEARCH, PHARMACEUTICAL SOCIETY OF KOREA, HEIDELBERG, vol. 35, no. 12, 21 December 2012 (2012-12-21), pages 2135-2142, XP035152651, ISSN: 1976-3786, DOI: 10.1007/S12272-012-1210-8**

• **HARRY MARTIN ET AL: "A PPAR[gamma] ligand present in Actinidia fruit ( Actinidia chrysantha ) is identified as dilinolenoyl galactosyl glycerol", BIOSCIENCE REPORTS, vol. 230, no. 3, 15 May 2013 (2013-05-15), pages 151-394, XP055136185, ISSN: 0144-8463, DOI: 10.1083/jcb.200203048**

• **NAKAMURA SEIKOU ET AL: "Chemical Structures and Hepatoprotective Effects of Constituents from the Leaves of Salacia chinensis", CHEMICAL & PHARMACEUTICAL BULLETIN (TOKYO), vol. 59, no. 8, August 2011 (2011-08), pages 1020-1028, XP002728870, ISSN: 0009-2363**

• **DATABASE WPI Week 200872 Thomson Scientific, London, GB; AN 2008-M14572 XP002728871, & CN 101 152 340 A (JIANGSU ANIMAL HUSBANDRY & VETERINARY ME) 2 April 2008 (2008-04-02)**

• **MUN FEI YAM ET AL: "Antioxidant and Hepatoprotective Effects of Murdannia bracteata Methanol Extract", JOURNAL OF ACUPUNCTURE AND MERIDIAN STUDIES, vol. 3, no. 3, 1 September 2010 (2010-09-01), pages 197-202, XP055135979, ISSN: 2005-2901, DOI: 10.1016/S2005-2901(10)60036-2**

**Description**

**Field of the Invention**

The present invention relates to a galactolipids-enriched plant extract, especially relates to an extract from *crassocephalum rabens (Juss. Ex Jacq.) S. Moore*, *gynura divaricata subsp. formosana* and *murdannia bracteata (C. B. Clarke) J. K. Morton ex D. Y. Hong.* In particular, the present invention relates to the uses of the plant extract to produce compositions for use in preventing or treating acute fulminant hepatitis or sepsis or the non-medical use for skin-whitening.

**Description of the Related Art**

Recently, researches indicate that many natural ingredients from plants (phytochemicals) have various curative effects. For example, Phan van Kiem et al. published in their article in Archives of Pharmacal Research, Vol. 35, pages 2135-2142 that the galactolipids from *ficus microcarpa* can inhibit TNF-$\alpha$-induced IL-8 secretion and therefore can provide anti-inflammatory effects. WO 2008/105436 A1 further discloses that the full extract of *Crassocephalum crepidoides* is bioactive and has a tumor nectrosis factor- ($\alpha$) (TNF-$\alpha$) suppression effect, a prostaglandin synthase 2 (COX-2) suppression effect and an anti-inflammatory effect. WO 2012/021068 A1 discloses a composition for treating hyperlipidemia and/or hypercholesterolemia, comprising pyrrozilidine alcaloids and preferably whole, parts of and or an aqueous or ethanolic leeching from a plant of the family Asteraceae, such as Gynura divaricata DC, in combination with one or more other lipid- and/or cholesterol-reducing substances. Mun Fei Yam et al. teach in their publication in the Journal of Acupuncture and Meridian Studies, Vol. 3, pages 197-202 that a concentrated methanol extract from *Murdannia bracteata (MB)* possesses antioxidant and free radical scavenging activities, inhibits the elevation of ALT and AST serum levels and could therefore be used to protect the liver. Nakamura Seikou et al., Chemical and Pharmaceutical Bulletin, Vol. 59, pages1020-1028 refers to the chemical structures and ex vivo investigations of the hepaptoprotective effect of the constituents from a methanolic extract from the leaves from *Salacia chinensis.* The publication from Harry Martin et al. in Bioscience Reports, Vol. 230, pages 151f. discloses that dLGG is able to non-covalently bind to PPAR$\gamma$ which may contribute to anti-inflammatory effects of dLGG. Document CN 101 152 340 A further discloses a traditional Chinese medicine composition for poultry, comprising *Hypericum perforatum, Solanum dulcamara, Murdannia bracteata* and *Bidens,* wherein such composition has anti-inflammatory, hepatotropic, virucidic and antibacterial activity and is suitable for treatment of poultry virus and bacterial infections of poultry, such as duck viral hepatitis and newcastle disease, with high cure rate.

Hereinafter, there are three other common plants in Taiwan *crassocephalum rabens (Juss. Ex Jacq.) S. Moore, gynura divaricata subsp. formosana* and *murdannia bracteata (C. B. Clarke) J. K. Morton ex D. Y. Hong for* illustration.

*Crassocephalum rabens* (*Juss. Ex Jacq.) S. Moore* (hereinafter "CR") is an ordinary wild vegetable in Taiwan and has great fecundity and adaptivity. The CR belongs to *crassocephalum* genus of *axteraceae* family. It tastes like crown daisy and can be collected all over the year. Especially, the taste of the CR is delicious when it collected before blossoming. Medical effects of the CR are known for relieving fever, strengthening stomach, reducing anasarca, and treating stomach ache. In addition, documents US 2008/152737 A1 and a corresponding publication from C.-C. Hou et al. in Cancer Research, Vol. 67, pages 6907-6915 disclose an extract of *C*. rabens, compositions therewith and methods using the same for treating cancer and inflammatory disorders associated with the activity of NF-$\kappa$B and its downstream inflammatory mediators, NO, iNOS, COX-2 and PGE$_2$.

*Gynura divaricata subsp. formosana* (hereinafter "GD"), another name of white-red vegetable, is a medical plant belongs to *gynura* genus of *axteraceae* family. It is an endemic species at Taiwan growing at coastal region and sometimes at low-elevation mountain of Taiwan. Young stem and leaf of the GD that can be used for food are claimed to have effects for diminishing inflammation, relieving fever, detoxification, diuresis, lowering blood pressure etc.

*Murdannia bracteata* (*C. B. Clarke) J. K. Morton ex D. Y. Hong* (hereinafter "MB") that is a perennial herb belongs to *monocotyledons* genus of *commelinaceae* family mostly grows at side of valley stream, valley water or sand land. It has medical values and is often used for reducing sputum, treating hemorrhoid or treating chronic infection diseases of neck lymph node, acute suppurative infection diseases of hair follicle, sebaceous gland or sweat gland.

However, recent studies are still limited about the active ingredients and the mechanisms of the foresaid three plants. There are no references or patents ever revealed an extract or a purified compound from the foresaid three plants has ability for treating or preventing indications such as hepatitis or septicaemia etc. and skin whitening.

Another way, structure of the galactoselipid, also called glycoglycerolipid, which largely exists at nature has two esterified fatty acids at *sn-1* and *sn*-2 position of glycerol and one to four galactoses at *sn*-3 position. Many natural or synthesis glycoglycerolipids are known for specific bioactivity, including antivirus, antitumor, anti-inflammation and immune depression etc. But this kind of compound is still unclear about its effects to other adaptations and molecular mechanisms of its bioactivity.

## SUMMARY OF THE INVENTION

According to deficient of the prior art, one purpose of the present invention is to provide a composition of pharmaceutical, healthy or nutritional additive for use in preventing or treating acute fulminant hepatitis by decreasing serum levels of aspartate aminotransferase (AST) and alanine aminotransferase (ALT), comprising a pharmaceutical, healthy or food acceptable vehicle, and an effective amount of a bioactive ingredient, wherein the bioactive ingredient is a galactolipids-enriched plant extract or a purified compound obtained from purification of the galactolipids-enriched plant extract, wherein the galactolipids-enriched plant extract is extracted from a plant sample by using water, methanol or ethanol and then using ethyl acetate to separate an ethyl acetate extract by partition, then further eluting the ethyl acetate extract by an alcohol elution buffer to have a galactolipids-enriched fraction, wherein the galactolipids-enriched fraction is a 1,2,di-O-$\alpha$-linolenoyl-3-O-$\beta$-galactopyranosyl-sn-glycerol (dLGG)-enriched fraction, wherein the plant sample selected from a group consisting of *gynura divaricata subsp. formosana*, *murdannia bracteata (C. B. Clarke) J. K. Morton ex D. Y. Hong,* and *crassocephalum rabens S. Moore,* and the alcohol is 5% to 20 % volume percentage of the alcohol elution buffer.

In an example of this present disclosure, the plant extract can further comprise a plant extract spectrum. For example, the plant extract is from *Gynura divaricata* subsp. *Formosana,* it can further comprise a chemical fingerprint of *gynura divaricata subsp. formosana.* The plant extract is from *murdannia bracteata (C. B. Clarke) J. K. Morton ex D. Y. Hong,* it can further comprise a chemical fingerprint of *murdannia bracteata (C. B. Clarke) J. K. Morton ex D. Y. Hong.*

In one example of this invention, the ratio of the alcohol in the alcohol elution buffer is about 5%~15 %. In another example of this invention, the ratio of the alcohol in the alcohol elution buffer is about 5%~10 %. In another example of this invention, the ratio of the alcohol in the alcohol elution buffer is about 10%~15 %.

In an embodiment of this present invention, the alcohol elution buffer can comprise dichloromethane and methanol, methanol and water, methanol and acetonitrile, or methanol and acetone.

In an example of this invention, the ratio of dichloromethane and methanol is 9:1 in a methanol elution buffer. The methanol elution buffer with the ratio of 9:1 can be used for elution, for example: to have a lower ether extract from *gynura divaricata subsp. formosana.*

In an example of this invention, the ratio of dichloromethane and methanol is 12:1 in the methanol elution buffer. The methanol elution buffer with the ratio of 12:1 can be used for elution, for example: to have a lower ether extract from *murdannia bracteata (C. B. Clarke) J. K. Morton ex D. Y. Hong.*

In an embodiment of this present invention, the galactolipids-enriched fraction can be further purified by a reversed phase high performance liquid chromatography to have a bioactive fraction.

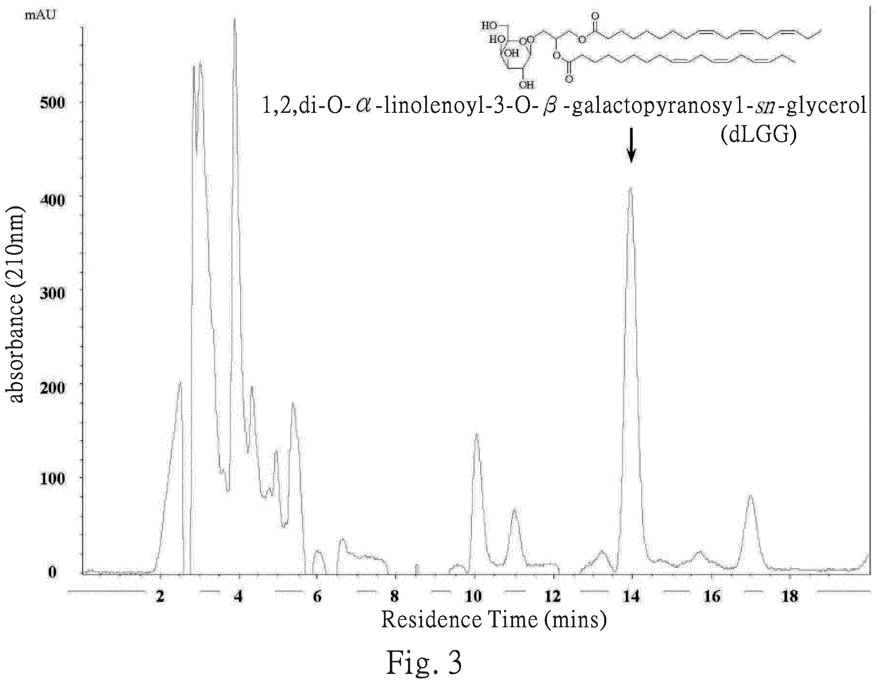

The bioactive fraction can comprise, a reversed phase high performance liquid chromatography spectrum (HPLC) of *crassocephalum rabens S. Moore* shown as Fig. 3.

In an embodiment of this present invention, the galactolipids-enriched fraction can be eluted by an alcohol elution buffer to have the bioactive fraction, wherein the alcohol is 100% to 70 % volume percentage of the alcohol elution buffer.

In this present invention, the galactolipids-enriched fraction is a 1,2,di-*O*-$\alpha$-linolenoyl- 3-*O*-$\beta$-galactopyranosyl-*sn*-glycerol (dLGG)-enriched fraction.

In this present invention, the purified object is 1,2,di-*O*-$\alpha$-linolenoyl-3-*O*-$\beta$-galactopyranosyl-*sn*-glycerol (dLGG).

Another purpose of the present invention is to provide a composition of pharmaceutical, healthy or nutritional additive for use in preventing or treating sepsis and sepsis-induced acute kidney injury associated with increased expression of hypoxia inducible factor-1$\alpha$ (HIF-1$\alpha$), comprising:

an effective amount of a bioactive ingredient, wherein the bioactive ingredient is a galactolipids-enriched plant extract or a purified object thereof, wherein the galactolipids-enriched fraction is a 1,2,di-*O*-$\alpha$-linolenoyl-3-O-$\beta$-galactopyranosyl-sn-giycerol (dLGG)-enriched fraction; and
a pharmaceutical, healthy or food acceptable vehicle.

In an embodiment of this present invention, the purified object is 1,2,di-*O*-$\alpha$-linolenoyl-3-O-$\beta$-galactopyranosyl-*sn*-glycerol (dLGG).

Another purpose of the present invention is to provide a composition for the non-medical use of skin whitening, comprising:

an effective amount of a bioactive ingredient, wherein the bioactive ingredient is a galactolipids-enriched plant extract or a purified object thereof, wherein the galactolipids-enriched fraction is a 1,2,di-O-$\alpha$-linolenoyl-3-O-$\beta$-galactopyran-osyl-sn-glycerol (dLGG)-enriched fraction; and
a pharmaceutical, healthy or food acceptable vehicle.

In this present invention, the purified object is 1,2,di-*O*-α-linolenoyl-3-*O*-β-galactopyranosyl-*sn*-glycerol (dLGG).

## BRIEF DESCRIPTION OF THE DRAWINGS

FIG. 1 shows the chemical fingerprinting of the galactolipids-enriched fraction from *Gynura divaricata* subsp. *formosana.* (hereinafter "GDE"). dLGG was determined as a major component in the fraction., wherein:

(A) is total ion chromatogram of the bioactive fraction GDE.
(B) is the chemical structures of monogalactosyldiacylglycerol constituents (1-5) of GDE .

FIG. 2 shows the chemical fingerprinting of the galactolipids-enriched fraction from *Murdannia bracteata* (C. B. Clarke) *J. K. Morton ex D. Y. Hong* (hereinafter "MBE"). dLGG was determined as a major component in the fraction, wherein:

(A) is total ion chromatogram of the bioactive fraction MBE.
(B) is the chemical structures of monogalactosyldiacylglycerol constituents (1-7) of MBE.

FIG. 3 shows the HPLC spectrum of the ethyl acetate solution from *Crassocephalum rabens (Juss. Ex Jacq.) S. Moore* (hereinafter "CR-EA").
FIG. 4 shows the effectiveness data of the dLGG-enriched galactolipid fractions from *Murdannia bracteata (C. B. Clarke) J. K. Morton ex D. Y. Hong* (MBE) and *Gynura divaricata* Subsp. *Formosana* (GDE) for the use in treating liver injury induced by LPS/D-GalN in mice. It proved that the dLGG-enriched galactolipid fractions extracted from MB (MBE) and GD (GDE) are effective to reduce the hepatic injury and acute fulminant hepatitis in LPS/D-GalN-induced mice, wherein:

(A) shows the serum levels of alanine aminotransferase (ALT) in the groups from left to right of vehicle (-),LPS/D-GalN-challenged mice (LPS/D-GalN+), LPS/D-GalN-challenged mice with SM (LPS/D-GalN+, Post-SM+), LPS/D-GalN-challenged mice with plant extract MBE (LPS/D-GalN+, Post-MBE+), LPS/D-GalN-challenged mice with plant extract GDE (LPS/D-GalN+, Post-GDE+). Data are mean $\pm$ S.E.M. of results of 6 mice per treatment group. Different letters indicate the significant difference within the tested groups ($P < 0.05$).
(B) shows the H&E staining and histology of liver sections from treated mice.

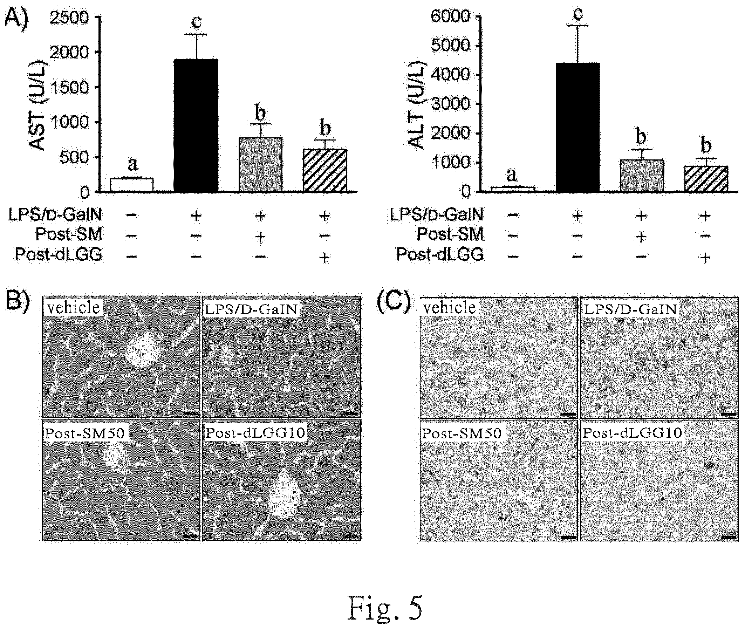

FIG. 5 shows the effectiveness of dLGG as a therapeutic agent for use in treating acute fulminant hepatitis of LPS/D-GalN-challenged mice, wherein:

(A) shows the serum levels of aspartate aminotransferase (AST) and ALT in the groups from left to right of vehicle (-), LPS/D-GalN-challenged mice (LPS/D-GalN+), LPS/D-GalN-challenged mice with SM (LPS/D-GalN+, Post-SM+), LPS/D-GalN-challenged mice with dLGG (LPS/D-GalN+, Post-dLGG+). Data are mean $\pm$ S.E.M. of results of 6 mice per treatment group. Different letters indicate the significant difference within the tested groups ($P < 0.05$).
(B) shows the H&E staining and histology of liver sections from treated mice. Representative image of each treatment group is shown.
(C) shows the result of TUNEL assay of liver tissues.. Representative image of each treatment group is shown. Brownish cells are TUNEL-positive apoptotic cells.

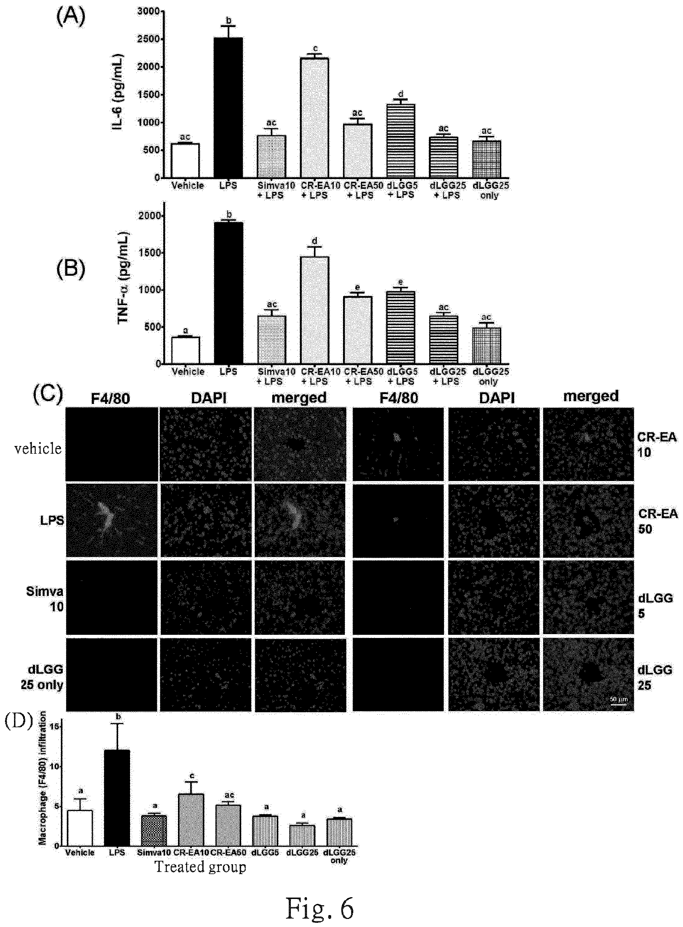

Fig. 6 shows the effectiveness of CR-EA, dLGG and simvastatin (simva) for use in preventing and treating LPS-induced inflammation and sepsis in mice. Data are mean $\pm$ SEM ($n$ = 4). Different letters indicate the significant difference within the tested groups ($P < 0.05$, ANOVA).

(A) shows the serum levels of IL-6 in the different treatment groups.
(B) shows the serum levels of TNF-α in the different treatment groups.
(C) shows F4/80 immunohistochemistry staining liver sections for illustrating infiltration of macrophage cells. Representative images are shown.
(D) shows a quantitative comparison among treatment groups determined by mean intensities of F4/80 positive cells.

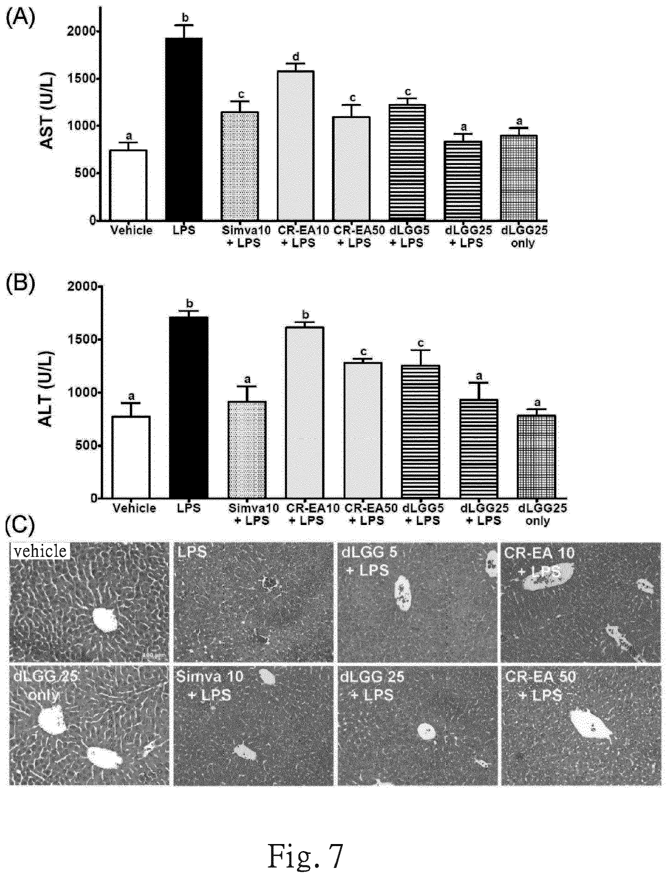

Fig. 7 shows the effectiveness of CR-EA, dLGG and simvastatin (simva) in the use for preventing and treating LPS-induced inflammation and sepsis in mice. Data are mean ± SEM ($n$ = 4). Different letters indicate the significant difference within the tested groups ($P < 0.05$, ANOVA).

(A) shows the serum levels of AST in the different treatment groups.
(B) shows the serum levels of ALT in the different treatment groups.
(C) shows the H&E stained liver sections. It illustrates the results of the liver tissue morphology and red blood cell and inflammatory cell infiltration in different treated groups of LPS-challenged mice. Representative images are shown.

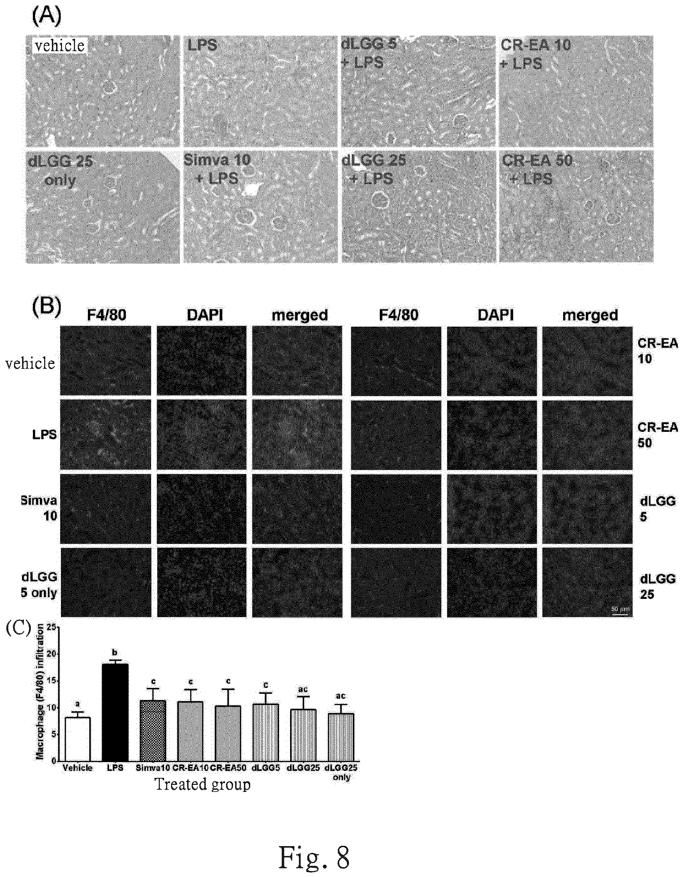

Fig. 8 shows the effectiveness of CR-EA, dLGG and simvastatin (simva) in the use for preventing and treating LPS-induced inflammation and sepsis in mice, wherein:

(A) shows renal histology in the different treatment groups of LPS-challenged mice by H&E-stained kidney sections. Representative images are shown.
(B) shows renal infiltration of macrophage cells by F4/80 immunohistochemistry staining of kidney sections. Representative images are shown.
(C) shows a quantitative comparison among treatment groups determined by mean intensities of F4/80 positive cells.

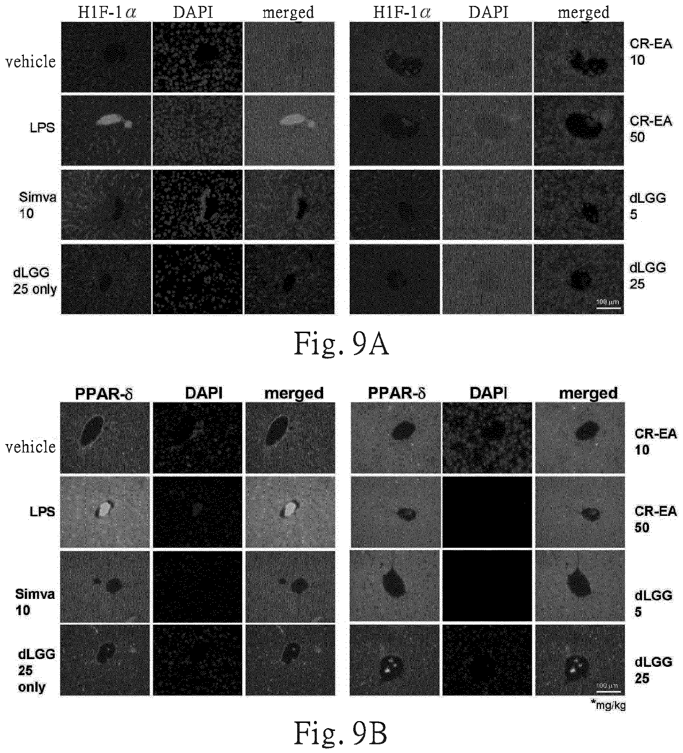

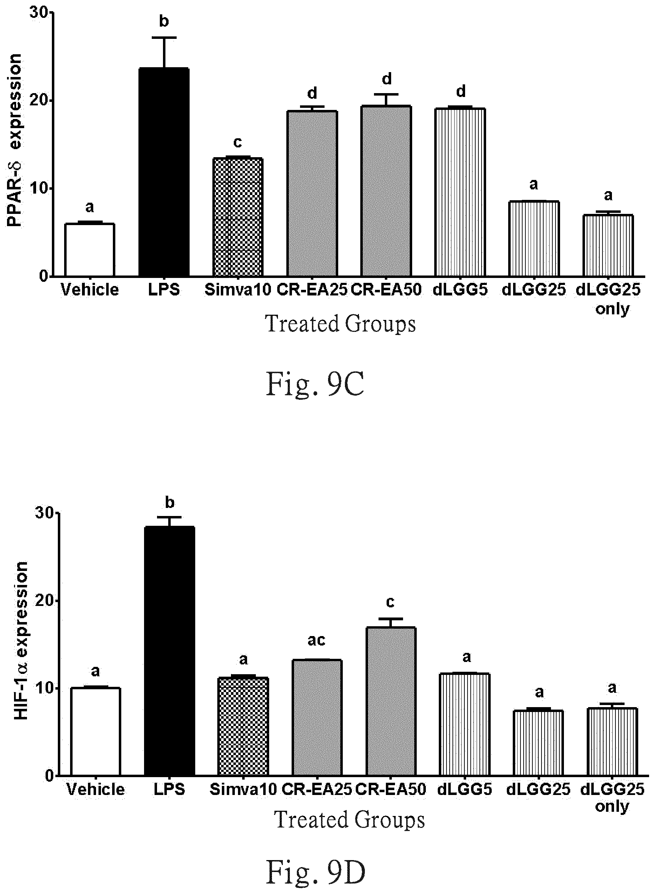

Fig. 9 shows the effectiveness of CR-EA, dLGG and simvastatin (simva) in the use for reducing the expression of proteins involved in the induction of hypoxia and production of lipid mediators related to inflammation and sepsis, wherein:

(A) shows the increasing expression of hypoxia inducible factor-1α (HIF-1α) attenuated by CR-EA, dLGG and simvastatin treatments. Representative images are shown.
(B) demonstrated the expression of PPAR-δ decreased by CR-EA, dLGG and simvastatin treatments. Representative images are shown.
(C) and (D) show quantitative comparisons among treatment groups determined by mean intensities of HIF-1α and PPAR-δ expression levels, respectively.

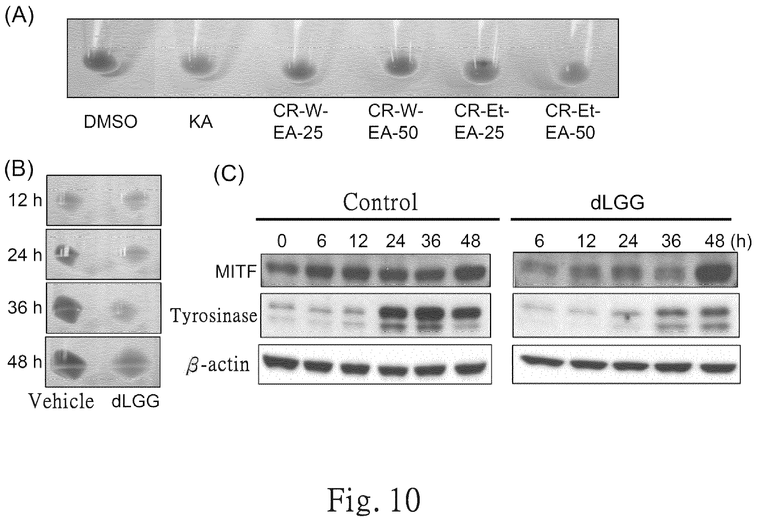

Fig. 10 shows the depigmentation effect of CR-EA or isolated single compound of dLGG from *Crassocephalum rabens,* wherein:

(A) shows the photographs of pellets of B16 melanoma cells after 72 h treatment by 50 μg/mL of kojic acid (KA) or EA fractions derived from total boiling water extracts (CR-W-EA) or total ethanolic extracts (CR-Et-EA) at 25 μg/mL or 50 μg/mL.
(B) shows depigmentation in B16 melanoma cells by 45 μM dLGG at different time points..
(C) shows western blot analysis of the expression of melanogenesis associated protein MITF and tyrosinase with vehicle (control group) or 45 μM dLGG treatment.

## DETAILED DESCRIPTION OF THE INVENTION

The term "effective amount, effective amount for preventing and/or treating" means the amount (weight percentage of composition) of the bioactive ingredient (extract or compound) for generating specific effect, preventing and/or treating effect. The person skilled in the art related to this present invention should understand that the effective amount can be different because of reasons such as trying to reach specific effect, to prevent and/or treat the kind of diseases and the way to deliver drugs. For generally, the amount of the bioactive ingredient in compound can be about 1% to about 100% of the weight of the composition, better is about 30% to 100%.

The term "medical, healthy or food acceptable vehicle" includes any standard medical, healthy or food acceptable vehicle. The vehicle that can be solid or liquid depends on the form of pharmaceutical, nutritional addictive or healthy composition. Examples of the solid vehicle include lactose, sucrose, gelatin and agar. Examples of the liquid vehicle include normal saline, buffered saline, water, glycerol and methanol.

The term "purified object" means a purified product from any purification process of a source or a crude product (ex. the plant extract in this present invention).

Embodiment of this present invention is further described with the following examples. The purposes, features and advantages of this present invention will become more clarify because of the following description and figures.

1. Materials and methods

1.1 Reagents and antibodies

D-galactosamine N (D-GalN), lipopolysaccharide (LPS), silymarin, simvastatin, kojic acid (KA) and dimethyl sulfoxide (DMSO) were purchased from Sigma Chemical Co. (St. Louis, MO). Prestained protein markers (Bioman, Taipei, Taiwan) were used to estimate the molecular weight markers of SDS-PAGE. The example of this present invention also used the primary antibodies against tyrosinase, microphthalmia-associated transcription factor (MITF) (Santa Cruz Biotechnology) and F4/80 antibody (eBioscience). Recombinant mice TNT-$\alpha$, and ELISA kit for measuring TNT-$\alpha$ and IL-6 were from R&D Systems, Inc. (Minneapolis, MN). Commercial kits purchased from Randox Laboratories (UK) were used to test the activity of serum aspartate aminotransferase (AST) and alanine aminotransferase (ALT). All other chemicals and solvents were of reagent or high-pressure liquid chromatography (HPLC) grade.

1.2 Cell culture

B16 melanoma cell line was obtained from ATCC (Manassas, VA) and grown in RPMI medium 1640 (Gibco/BRL) supplemented with 10% heat-inactivated fetal bovine serum, 100 unit/mL penicillin and 100 $\mu$g/mL streptomycin, at 37°C in a humidified 5% $CO_2$ incubator.

1.3 *Depigmentation assay*

B16 melanoma cell line was plated on to 10 cm culture dishes at an initial density of $1 \times 10^5$ cells dish$^{-1}$ and then cultured under the same condition described above. After 12 hours of seeding, the cells were treated by vehicle DMSO, kojic acid (KA), ethyl acetate (EA) fractions of CR's water and ethanolic extractions (Hou et al., 2007) for 72 hours. These cells were harvested by centrifuge and visually evaluated the color of these cell pellets. In another experiment, depigmentation effect of the single compound dLGG was observed and evaluated at different time points with the same procedure.

1.4 Animals

Female C57BL/6J mouse or female ICR mouse (4-week-old) were supplied from National Laboratory Animal center (Taipei, Taiwan) and given a standard laboratory diet and distilled water *ad libitum* and kept on a 12-hours light/dark cycle at $22 \pm 2$°C. The embodiment in this present invention was proceed following the institutional guideline and approved by the Institutional Animal Care and Utilization Committee of Academia Sinica, Taiwan.

1.5 Plant extracts and dLGG preparation

Preparation of extracts from CR, MB, and GD were followed the protocol previously published by Hou et al. (Hou et. al, Cancer Research 67, 6907-6915, 2007) with some modifications. About 15 kg of fresh whole plant was extracted by 2-3 times weight of 95% ethanol at room temperature. Ethyl acetate was used to partition total ethanolic extracts to produce the ethyl acetate (EA) fraction (GD: 8.6 kg; MB: 8.9 kg). Furthermore, dichloromethane-methanol was used to eluting a silica gel column for separating the EA fraction of GD and MB for yielding 10 subfractions respectively, wherein the elution ratio of methanol to dichloromethane for GD was 1:9, for MB was 1:12 and for CR was 1:9. For GD, subfraction 7 (817 g) was further purified using a Diaion HP-20 gel column eluted with 95% ethanol to give a monogalactosyldiacyl glycerols-enriched fraction (designated GDE; 253.3 g). For MB, subfraction 6 (1.06 kg) was further purified using a Diaion HP-20 gel column eluted with 95% ethanol to give a monogalactosyldiacyl glycerols-enriched fraction (designated MBE; 360.1 g).

RP-HPLC/APCI-M was used to determine the chemical fingerprinting of the enriched fractions. Compound characterization was done using $^1$H and $^{13}$C nuclear magnetic resonance (NMR) together with APCI/MS, using a Bruker ADVANCE 500 AV NMR spectrometer and Thermo Finnigan/LCQ Advantage mass spectrometer running in positive ion mode, respectively.

And then, reversed phase HPLC was conducted under the following column and condition to have CR-EA fraction and chemical spectrum thereof. The column was semi-preparative column (Phenomenex 5 $\mu$m, C18, 250×4.6 mm) and the condition was to carry on isocratic elution by 98% ethanol, 1 mL/min velocity of flow. The dLGG shown at HPLC spectrums were taken as an indicated compound of bioactive EA fractions.

GDE's or MBE's HPLC spectrum could be done through the same protocol to treat galactolipids-enriched fractions of GDE or MBE (eluting it by methanol-dichloromethane, wherein the ratio of ethanol to dichloromethane was about 9:1 to 8:2).

1.6 Protection effects of dLGG or dLGG containing plant extracts on acute fulminant hepatitis in mice-induced by LPS/D-GalN

The hepatoprotective effect in live body of compound dLGG isolated from *Crassocephalum rabens* (Asteraceae) in LPS/D-GalN-induced fulminant hepatitis were investigated and compared with the hepatoprotective drug, silymarin (SM). Mice were randomly separated into four groups (n=6 per group) for treatments: vehicle; LPS/D-GalN; 50 mg/kg of silymarin (Post-SM50); and 10 mg/kg dLGG (post-dLGG10), and all treatments were given intraperitoneally. One hour after the mice treated with 500 ng LPS and 250 $\mu$L saline containing 25 mg D-GalN (Huang et al., 2012), the dLGG and silymarin were given intraperitoneally. Besides, the other two groups of mice were treated dLGG or silymarin continuously for three days to perform the protection effect of dLGG against LPS/D-GalN induced fulminate hepatitis. Eight hours after LPS/D-GalN injection, blood samples were collected through retro-orbital bleeding, and then all mice were sacrificed to collect blood samples and liver tissues.

1.7 Protection or treatment effect of dLGG or dLGG containing plant extract on sepsis in t mice-induced by LPS

The therapeutic effect of dLGG or dLGG containing CR extracts was estimated by inducing acute inflammation and septic shock using LPS, and the simvastatin (Simva) was used as positive control group. Mice were randomly separated into different treatment groups: vehicle, 10 mg/kg LPS, 10 mg/kg simvastatin (Simva 10), 10 mg/kg CR-EA extraction (CR-EA 10), 50 mg/kg CR-EA extract (CR-EA 50), 5 mg/kg dLGG (dLGG 5) and 25 mg/kg dLGG (dLGG 25). The plant components or the drugs were all injected an hour before administration of LPS. Another animal group was only treated with 25 mg/kg dLGG (dLGG 25 only). All groups were sacrificed after 24 hours and blood samples were collected by retro-orbital bleeding before scarification of mice and organ tissues were collected immediately.

1.8 Histology and immunohistochemistry method

Liver, lung and kidney tissues were fixed in 10% buffered formalin then it were embedded into paraffin. The paraffin-embedded tissues were sliced (8-$\mu$m) and stained by hematoxylin and eosin (H&E). Besides, sections of paraffin-embedded liver and lung (4-$\mu$m for thickness) were heat immobilized and deparaffinized by use of xylene and rehydrated in a graded series of ethanol with a final wash in distilled water, and finally soaked into decloaking chamber (Biocare Medical) containing Target Retrieval Solution (DakoCytomation) for antigen retrieval.
*In situ* detection of apoptotic cells was conducted according to the manufacturer's protocol (Chemicon). It is using terminal deoxynucleotidyl transferase to work on nucleotide sequence to form a nick at dUTP position for terminal deoxynucleotidyl transferase-mediated dUTP nick end labeling. Finally, AxioVision software (Carl Zeiss MicroImaging, Inc.) was used to analyze the number of TUNEL-positive cells.
Immunohistochemistry with liver, lung and kidney tissues were done by incubating the samples into F4/80 primary antibody for overnight respectively. After subsequent washing, each of the samples was incubated using florescent-labeled secondary antibody. Florescent images of the macrophage cell infiltration (positive F4/80 stained cell) were captured by AxioVision software (Carl Zeiss MicroImaging, Inc.) and then analyzed.

1.9 Western blot

The liver tissue (0.1 g) of each mouse was estimated by homogenization in a mixer ball mill (MM301, Retsch, Haan, Germany) for 2 minutes, extracted by adding 0.4 mL lysis buffer and centrifuged at 15,000xg for 30 min at 4°C (Shyur et al., 2008). The supernatant was collected, and total protein concentration of the sample was determined by DC protein test kit (Bio-rad). Protein was resolved by 5%~20% gradient SDS-PAGE and then transferred to membrane and immunoblotted with enhanced chemiluminescence reagents (ECL, Amersham) and monoclonal antibodies against specific protein.
The cellular proteins were produced according to previously published method (Chiang et al., 2005). Protein content was measured by Bradford method (Bio-Rad).

1.10 Measurement of serum AST and ALT activity

Blood samples of tested mice were centrifuged under 4°C, 1400 $\times$ g for 15 minutes to separate serum from blood. Commercial kit purchased from Randox Laboratories (UK) was used to determine the activity of aspartate aminotransferase (AST) and alanine aminotransferase (ALT) in serum supernatants.

1.11 Measurement of serum IL-6 and TNF-$\alpha$

Serum levels of interleukin (IL-6) and tumor necrosis factor-alpha (TNF-$\alpha$) were determined using a commercial kit from eBioscience.

1.12 Statistical analysis

All data are expressed as means $\pm$ standard error of the mean (SEM). Differences were compared by ANOVA. Different letter superscripts indicate significant difference within treatments; $P < 0.05$ was considered statistically significant.

2. Results

2.1 Chemical fingerprints for three food or pharmaceutical plant of *Gynura divaricata* subsp. *formosana* (Asteracae) and *Murdannia bracteata* (C. B. Clarke) (Commelinaceae)

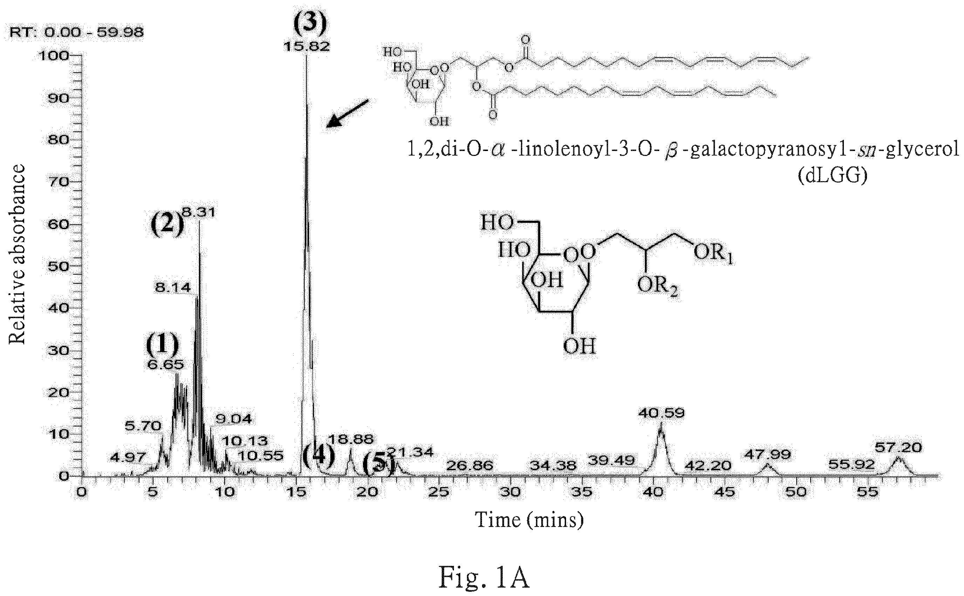

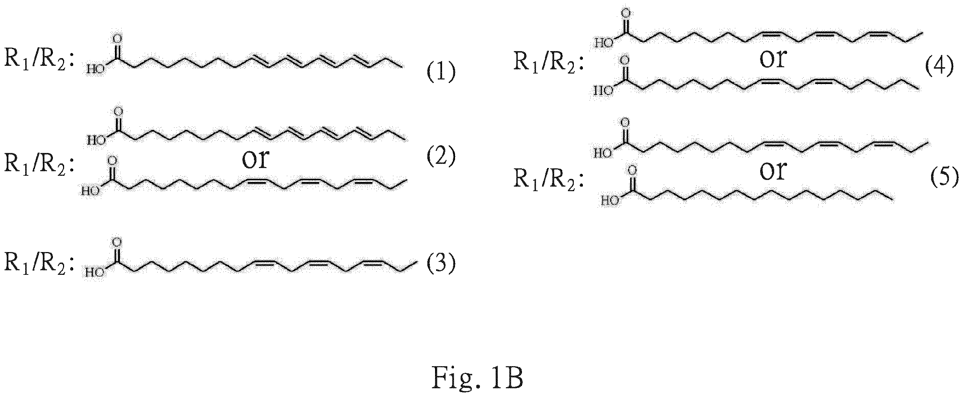

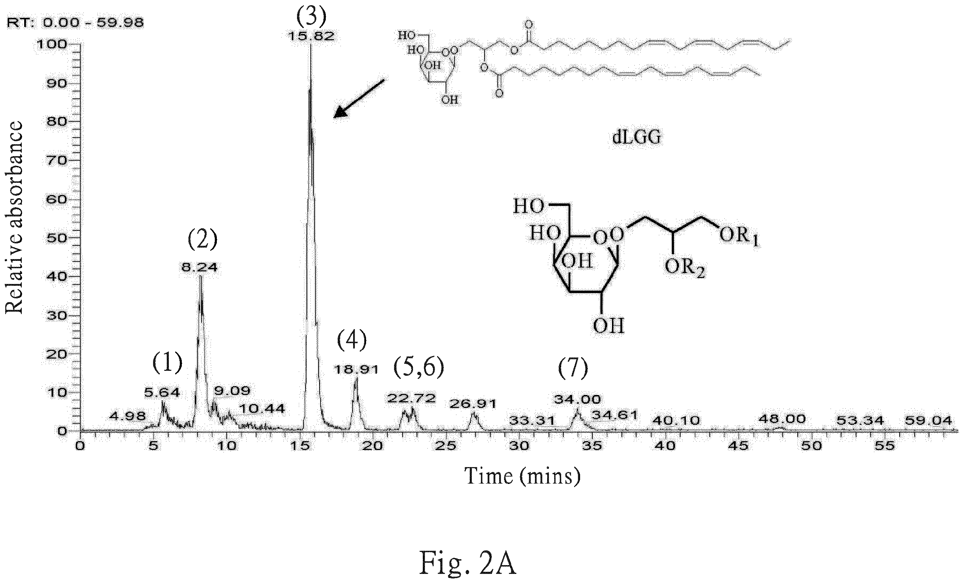

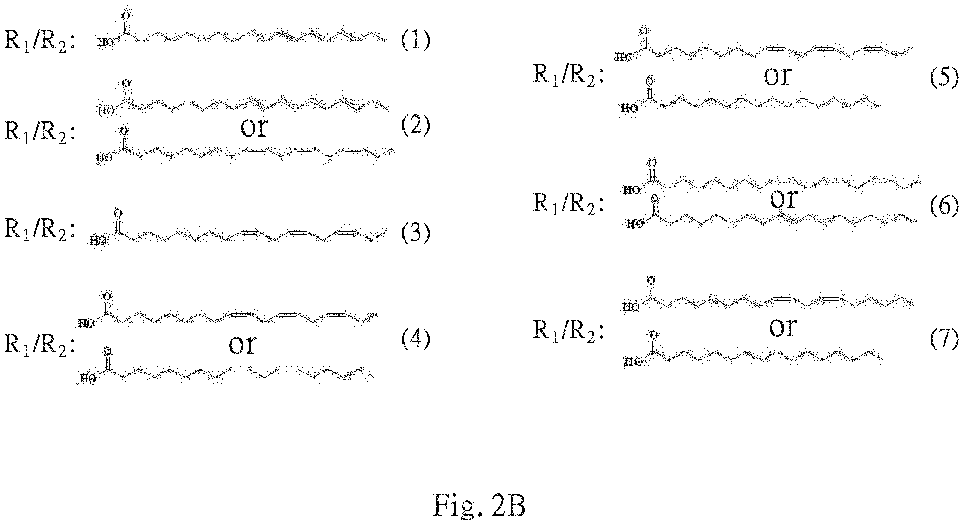

RP-HPLC and atmospheric chemical ionization-mass spectrometry (ACPI-MS) were used to establish the chemical fingerprints of bioactive fraction GDE and MBE. All monogalactosyldiacylglycerol constituents present in the galactolipids-enriched fractions of GDE and MBE were identified. As shown in figure 1 and table 1, 1,2,di-*O*-$\alpha$-linolenoyl-3-*O*-$\beta$-galacto pyranosyl-*sn*-glycerol (dLGG) was identified the major component of CD. As shown in figure 2 and table 2, dLGG was identified the major component of MB. The chemical fingerprint of CR-EA, shown in figure 3, was taken by RP-HPLC, wherein dLGG was the indication compound in the bioactive EA fraction (CR-EA) of CR.

Fig. 1A and fig. 2A show the compound peak distribution on the total ion chromatogram of GDE and MBE respectively. Five galactolipid compounds were found in GDE with the fatty acid moieties identified as 18:3/18:3 (dLGG), 18:4/18:4 (1), 18:4/18:3 (2), 18:2/18:3 (4), 16:0/18:3 (5). Meanwhile, the fatty acid moieties of the seven galactolipid compounds in MBE were identified as 18:3/18:3 (dLGG), 18:4/18:4 (1), 18:4/18:3 (2), 18:2/18:3 (4), 16:0/18:3 (5), 18:1/18:3 (6), and 16:0/18:2 (7). It is not confirmed that the *sn* positioning of the two fatty acid moieties in the compounds 2, 4, 5, 6 and 7.

The *m/z* of the Na$^+$ adduct [M + Na]$^+$ and fragment ions corresponding to the diacylglycerol, monoacylglycerol, and fatty acid moieties from ACPI-MS are tabulated in tables 1 and 2. Herein, the meanings of abbreviations in tables 1 and 2 are as the following: 18:3, $\alpha$-Linolenic Acid; 18:2, $\alpha$-Linoleic acid; 18:1, Oleic acid; 16:0, palmitic acid. The peak percentage was calculated by the following formula:

$$\% = \text{each peak area } / \text{ peak area of total monogalactosyldiacylglycerol compounds } \times 100\%$$

Table 1: APCI-MS data of five major monogalactosyl diglyceride compounds in GDE fraction

| Ion peak | peak | | | | |
|---|---|---|---|---|---|
| | dLGG(3) | 1 | 2 | 4 | 5 |
| *m/z* [M + Na]$^+$ | 797(4) | 793(1) | 771(6) | 799(3) | 738(1) |
| Diacylglycerol moieties | | | | | |
| [CH$_2$(OCOR$_1$)CH(OCOR$_2$)-CH$_2$OH$_2$]$^+$ | 613(100) | 609(100) | 611(100) | 615(100) | 554(100) |
| [CH$_2$(OCOR$_1$)CH(OCOR$_2$)-CH$_2$]$^+$ | 595(13) | 591(43) | 613(13) | 597(13) | 536(15) |
| Monoacylglycerol moieties | | | | | |
| [CH$_2$(OCOR$_1$)CH(OH)CH$_2$]$^+$ | 335(3) | 334(3) | 335(3) | 337(4) | 364(11) |
| [CH$_2$(OH)CH(OCOR$_2$)CH$_2$]$^+$ | | | 337(4) | 335(5) | 391(3) |
| Acyl moieties | | | | | |
| [R$_1$]$^+$ and [R$_2$]$^+$ | 261(23) | 259(30) | 261(32) | 263(24) | 239(22) |
| | | | 260(26) | 261(26) | 261(17) |

(continued)

| Acyl moieties | | | | | |
|---|---|---|---|---|---|
| Residence time (min) | 15.82 | 6.65 | 8.31 | 18.88 | 21.34 |
| Molecular species (fatty acid/ fatty acid) | 18:3/18:3 | 18:4/18:4 | 16:0/18:2 | 18:2/18:3 | 16:0/18:3 |
| % (peak percentage) | 88.1 | 3.2 | 7.5 | 1.0 | 0.1 |

Table 2: APCI-MS data of five major monogalactosyl diglyceride compounds in MBE fraction.

| Ion peak | peak | | | | | | |
|---|---|---|---|---|---|---|---|
| | dLGG(3) | 1 | 2 | 4 | 5 | 6 | 7 |
| $m/z$ [M + Na]$^+$ | 797(1) | 793(1) | 771(5) | 799(3) | 775(4) | 801(5) | 777(10) |
| Diacylglycerol moieties | | | | | | | |
| $[CH_2(OCOR_1)CH-(OCOR_2)CH_2OH_2]^+$ | 613 (100) | 609 (100) | 611 (100) | 615 (100) | 591 (23) | 617 (100) | 575 (100) |
| $[CH_2(OCOR_1)CH-(OCOR_2)CH_2]^+$ | 595 (11) | 591 (14) | 613 (6) | 597 (9) | 573 (7) | 599 (30) | 593 (6) |
| Monoacylglycerol moieties | | | | | | | |
| $[CH_2(OCOR_1)CH-(OH)CH_2]^+$ | 335(5) | 334(3) | 335(5) | 337(3) | 313(9) | 339(10) | 313(24) |
| $[CH_2(OH)CH-(OCOR_2)CH_2]^+$ | | | 337(7) | 335(3) | 335(4) | 335(8) | 337(10) |
| Acyl moieties | | | | | | | |
| $[R_1]^+$ and $[R_2]^+$ | 261 (22) | 259 (24) | 261 (29) | 263 (21) | 239 (22) | 265 (10) | 239 (21) |
| | | | 260 (21) | 261 (17) | 261 (15) | 261 (16) | 263 (30) |
| Residence time (min) | 15.82 | 5.64 | 8.24 | 18.91 | 22.72 | 22.72 | 26.91 |
| Molecular species (fatty acid/ fatty acid) | 18:3/18:3 | 18:4/18:4 | 16:0/18:2 | 18:2/18:3 | 16:0/18:3 | 18:1/18:3 | 16:0/18:2 |
| % (peak percentage) | 78.9 | 1.9 | 12.2 | 2.2 | 1.2 | 1.2 | 2.4 |

Fig. 1B and fig. 2B show the chemical structures of monogalactosyldiacylglycerols in GDE and MBE fractions respectively. The major and active constituent compound is dLGG with the enriched fractions containing 88.1% (*G. divaricata Subsp. Formosana*), and 78.9% (*M. bracteata*). And dLGG was determined as 65.7% in the bioactive fraction of *C. rabens* (Hou et al., 2007). With dLGG as an indication compound of galactolipids-enriched fraction extracted from the three pharmaceutical plants in this present invention, the developed chemical fingerprint profiling method can be used to ensure the consistency of he batch-to-batch preparations of the bioactive fractions and can be the protocol for regular quality assurance.

2.2 dLGG as a therapeutic agent for acute fulminant hepatitis of LPS/D-GaIN induced mice

Fulminant hepatic failure (FHF), synonymous with acute liver failure, is related to severe liver disorders that results in rapid distortion of hepatic function that often leads to devastating consequences (Sass and Shakil, 2005). It is a life-threatening disease, with orthotopic liver transplantation as the only curative treatment at present (Russo & Parola, 2011).

LPS/D-GaIN induced mice to cause acute fulminant hepatitis can be a widely used animal model to mimic the cascaded events of FHF observed in clinic (Kosai et al, 1999). This present invention uses this model to evaluate the therapeutic effect of dLGG, a bioactive compound isolated from MB, CR and GD, and the dLGG-enriched galactolipid fractions GDE and MBE on liver injury.

At the same time, a commercial hepatoprotective drug, silymarin (SM), was used as a reference control. Mice were given LPS/D-GalN 1 hour before intraperitoneal injection of vehicle control 0.5% DMSO, dLGG (10 mg/kg) and SM (50 mg/kg). Mice blood sera from different groups were collected to measure the two clinical indicators of hepatic injury or dysfunction, aspartate aminotransferase (AST) and alanine aminotransferase (ALT) activities.

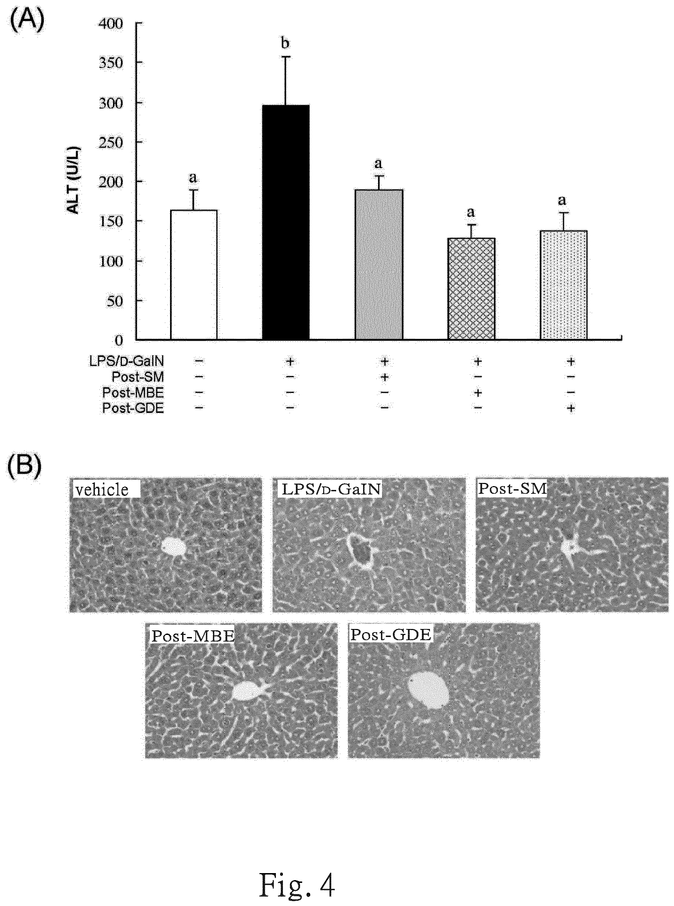

Fig. 4 and fig. 5 show the activity assessment result of galactolipids-enriched fraction from GDE and MBE, and pure compound of dLGG. According to fig 4A, it shows that GDE, MBE and the hepatoprotective drug SM can effectively reduce the two-fold increase of serum ALT levels in LPS/D-GalN-challenged mouse compared to the control group.

As shown in fig. 4B, histopathology examination by H&E staining showed that the observed infiltration of inflammatory cells into liver lobules, tissue destruction and erythrocyte influx of liver tissue etc. in LPD/ D -GalN challenged mice were significantly decreased in the treated groups.

As shown in fig. 5, it shows that treatment with dLGG after induction of fulminant hepatitis can significantly inhibit the increased levels of serum AST and ALT induced by LPS/D-GalN in mice, as did by SM. Furthermore, fig. 5 shows that LPS/D-GalN-challenged mice liver exhibited infiltration of inflammatory cells into liver lobules, tissue destruction and erythrocyte influx as compared to the vehicle control, and which was attenuated by both dLGG and SM treatment Moreover, dLGG treatment also significantly decreased the numbers of apoptosis cells, TUNEL positive-stained cells in, in liver tissue of mice.

Besides, dLGG still have significant protection effect in mice pre-treated with dLGG (1 or 10 mg/kg) 1 hour before LPS/D-GalN-challenge (data not shown). These results indicate that dLGG has therapeutic and protective effect against LPS/D-GalN-induced fulminant hepatic failure.

2.3 Protective and therapeutic effect of galactolipids-enriched *C. rabens* extract and dLGG against LPS-induced inflammation and sepsis in mice

Acute inflammation will cause serious systemic response including sepsis. Sepsis is a complicate indication that causes rapid organ failure and death. Acute kidney injury (AKI) is a common life-threatening disease that has about 45% mortality in past thirty years, and about half of patients of these severe AKI are triggered by sepsis (Uchino et al., 2005; Yasuda et al., 2006).

Endotoxin is an outer membrane component of Gram-negative bacteria which can induce a serious inflammatory cascade reaction and involve in the pathogenesis of sepsis. Inflammation model constructed by LPS infusion/injection has been widely used to study sepsis (Doi et al., 2009). In the embodiment of this present invention, LPS was used to induce acute inflammation and septic shock to observe and evaluate the protective and therapeutic effect of dLGG (5 and 25 mg/kg weight) and CR-EA (10 and 50 mg/kg weight) by patho-physiological comparison of liver, kidney and lung, by determining the serum levels of inflammatory cytokines, IL-6 and TNF-$\alpha$, and ALT and AST, indicators of liver damage and hepatotoxicity, and by comparison for the expression levels of hypoxia inducible factor-1$\alpha$ (HIF-1$\alpha$) and peroxisome-proliferator activating receptor $\delta$ (PPAR-$\delta$) involved in the induction of hypoxia and in the production of inflammatory lipid mediators among the treatment groups.

An hour before LPS administration, 0.5% DMSO was treated to control group and 10 mg/kg simvastatin, dLGG and CR-EA were treated to positive control group. Simvastatin, a clinical drug, is a HMG-CoA reductase inhibitor and has clinically beneficial effect on cardiovascular, cerebrovascular and acute and chronic kidney diseases (Epstein et al., 2005; Nissen et al., 2005; Almog, et al., 2004). Statin therapy (statin is a category of HMG-CoA reductase inhibitor drug, wherein simvastatin is one of the category) has effect for preventing sepsis and sepsis-induced acute kidney injury of human and animal (Yasuda et al., 2006). Moreover, one group of mice was treated with dLGG (25 mg/kg) without LPS challenge. All the mice were sacrificed after 24 hours. Mice sera were collected to measure and compare the concentration of IL-6 and TNF-$\alpha$, and the levels of AST and ALT.

Fig.6A, fig. 6B, fig. 7A and fig. 7B show that the levels of inflammatory stimulation cytokines in serum significantly decreases in the mice groups treated with CR-EA and dLGG at high dose, respectively, and then by LPS-challenge, wherein after CR-EA and dLGG treated, IL-6 significantly decreases 2.6 and 3.5 times respectively, TNF-$\alpha$ decreases 2.0 and 3.0 times respectively, ALT decreases 1.75 and 2.3 times respectively, and AST decreases 1.3 and 1.8 times respectively.

As shown in figures 7C, histology comparison of H&E staining between the different treated groups reveals that the inflammatory cells or red blood cell infiltration in liver of mice was induced by LPS-challenge. Fig. 8A shows the effect of CR-EA and dLGG pre-treated for preventing acute kidney injury. H&E staining result can be observed damage of adrenal cortex, vacuolar degeneration of tubular cells and macrophage infiltration. It has been reported that simvastatin can improve sepsis-induced acute kidney injury and mortality using cecal ligation and puncture operation in mice (Yasuda et al., 2006). The embodiment of this present invention, fig. 5A can be observed that simvastatin can prevent sepsis-induced acute kidney injury of LPS treated mice, and the same remarkably protective effect can be observed in the CR-EA or dLGG treated group. These effects can be concluded from renal histology of the CR-EA or dLGG-treated group is similar to those of the vehicle control group mice. F4/80 immunohistology staining results show that treatment with CR-EA or dLGG resulted to decreased infiltration and activation of macrophages in liver, as shown in fig. 8C, in comparison with the vehicle control group. The positive control group (Simva 10) shows similar result in comparison with the CR-EA or dLGG treated group. Furthermore, after LPS-challenge, treatment of dLGG at dose of 25 mg/kg dose can effectively reduce damages in mice liver, lung and kidney caused by LPS (data not shown).

Fig. 9 further demonstrated that pre-treatment of CR-EA and dLGG will down-regulated the expression of two major proteins, PPAR-$\delta$ and HIF-1$\alpha$, which involve in the formation of inflammatory mediators and induction of hypoxia. These results imply that CR-EA and dLGG treatment can attenuate organ damage caused by oxygen deprivation and hypoxia conditions and may contribute to the regulation of inflammatory lipid mediators in homeostatic levels.

According to figures 6 to 9, it shows that the levels of IL-6, TNF-$\alpha$, AST and ALT in the sera, pathological evaluations in organ tissues, such as liver and kidney, and expression levels of several protein markers from dLGG treated only mice were similar to those from the vehicle treated mice, as well as lung analysis result (data not shown). These results illustrate that dLGG is harmfulless to mice.

In conclusion, these results show that CR extract and dLGG have potential to be preventive or therapeutic agent against LPS-induced sepsis.

2.4 Depigmentation B16 cells by dLGG and CR-EA extract

Skin, being the largest organ of body is always under the influence of internal and external factors. The skin reacts to those stimuli by modifying the constitutive pigmentation pattern. Melanin, a kind of pigment, is responsible for skin color and preventing skin damage or pigmentation induced from environment (ex. UV) or other factors (drugs or chemicals). Melanin is produced through the process of melanogenesis in melanocytes (Costin and Hearing, 2007). The enzyme tyrosinase e is essential in the production of melanin in mammals (Hearing and Tsukamoto, 1991). Microphthalmia-associated transcription factor (MITF) involved in the regulation of melanocyte cell differentiation, pigmentation, proliferation and survival which is also a major transcriptional regulator of genes encoding melanogenic enzymes or proteins, such as tyrosinase, tyrosinase-related protein 1 and protein 2 (Yasumoto et al., 1997; Hasegawa et al., 2010).

A highly pigmented B16 melanoma cell has been used as an experiment model to e study depigmentation effect of compounds from 70's to date (Bang et al., 2013; Wrathall et al., 1973). In the embodiment of this present invention, the EA fractions derived from the total crude water (CR-W-EA) and ethanolic (CR-Et-EA) extracts of *Crassocephalum rabens* were used to examine their ability to depigment B16 cells by direct observation of the cell pellets after treatment. KA and DMSO were used as positive and vehicle control, respectively.

Fig. 10(A) shows that CR-Et-EA possesses dose-dependent effect on depigmentation of B16 melanoma cells compare with the vehicle control. The depigmentation effect at different time points of single compound dLGG purified from CR-Et-EA was further assessed. Fig. 10(B) shows that dLGG (45 $\mu$M) has significantly depigmentation in a time-dependent manner in B16 melanoma cells compared with the vehicle control group.

Molecular mechanism of dLGG depigmentation effect was further studied. Western blotting assay was preceded for testing the effect of dLGG on tyrosinase and MITF expression. As shown in fig. 10(C), it shows that dLGG can certainly inhibit the expression of melanogenesis associated protein, MITF and tyrosinase. The data indicate that dLGG inhibit expression of MITF and tyrosinase to suppress melanogenesis that resulted in depigmentation of in B16 melanoma cells, suggesting dLGG can be used as a new skin-whiting agent.

It should be understood that the above-mentioned detailed description and specific examples, while indicating preferred embodiments of the invention, are given by way of illustration only.

**Claims**

1. A composition of pharmaceutical, healthy or nutritional additive for use in preventing or treating acute fulminant hepatitis by decreasing serum levels of aspartate aminotransferase (AST) and alanine aminotransferase (ALT), comprising:

   a pharmaceutical, healthy or food acceptable vehicle, and
   an effective amount of a bioactive ingredient, wherein the bioactive ingredient is a galactolipids-enriched plant extract or a purified compound obtained from purification of the galactolipids-enriched plant extract, wherein the galactolipids-enriched plant extract is obtained by the steps of:

   (a) using water, methanol or ethanol to extract a plant sample, wherein the plant sample is selected from a group consisting of: *Gynura divaricata subsp. formosana, Murdannia bracteata* (*C. B. Clarke*) *J. K. Morton ex D. Y. Hong,* and *Crassocephalum rabens S. Moore;*
   (b) using ethyl acetate to partition the extract from step (a) to obtain an ethyl acetate extract;
   (c) using an alcohol elution buffer for eluting a galactolipids-enriched fraction from the ethyl acetate extract from step (b), wherein the alcohol is 5% to 20 % volume percentage of the alcohol elution buffer,

wherein the galactolipids-enriched fraction is a 1,2,di-O-α-linolenoyl-3-O-β-galactopyranosyl-sn-glycerol (dLGG)-enriched fraction.

2. The composition for use in preventing or treating acute fulminant hepatitis according to claim 1, wherein the galactolipids-enriched fraction obtained in step (c) is further purified by a reversed phase high performance liquid chromatography to have a purified compound, wherein the galactolipids-enriched fraction is eluted by an alcohol elution buffer and the alcohol is 70 % to 100% volume percentage of the alcohol elution buffer, wherein the purified compound is 1,2,di-O-α-linolenoyl-3-O-β-galactopyranosyl-sn-glycerol (dLGG).

3. The composition for use in preventing or treating acute fulminant hepatitis according to claim 1, wherein the alcohol elution buffer for use in step (c) comprises methanol and water, methanol and dichloromethane, methanol and acetonitrile or methanol and acetone.

4. The composition for use in preventing or treating acute fulminant hepatitis according to claim 3, wherein the alcohol elution buffer is used to elute an ethyl acetate extract from *Gynura divaricata subsp. formosana.*

5. The composition for use in preventing or treating acute fulminant hepatitis according to claim 3, wherein the alcohol elution buffer is used to elute an ethyl acetate extract from *Murdannia bracteata (C. B. Clarke) J. K. Morton ex D. Y. Hong.*

6. A composition of pharmaceutical, healthy or nutritional additive for use in preventing or treating symptoms of sepsis and sepsis-induced acute kidney injury associated with increased expression of hypoxia inducible factor-1α (HIF-1α), comprising:

a pharmaceutical, healthy or food acceptable vehicle, and
an effective amount of a bioactive ingredient, wherein the bioactive ingredient is a galactolipids-enriched plant extract or a purified compound obtained from purification of the galactolipids-enriched plant extract, wherein the galactolipids-enriched plant extract is obtained by the steps of:

(a) using water, methanol or ethanol to extract a plant sample, wherein the plant sample is selected from a group consisting of: *Gynura divaricata subsp. formosana, Murdannia bracteata (C. B. Clarke) J. K. Morton ex D. Y. Hong,* and *Crassocephalum rabens S. Moore;*
(b) using ethyl acetate to partition the extract from step (a) to obtain an ethyl acetate extract;
(c) using an alcohol elution buffer for eluting a galactolipids-enriched fraction from the ethyl acetate extract from step (b), wherein the alcohol is 5% to 20 % volume percentage of the alcohol elution buffer, and

wherein the galactolipids-enriched fraction is a 1,2,di-O-α-linolenoyl-3-O-β-galactopyranosyl-sn-glycerol (dLGG)-enriched fraction.

7. The composition for use in preventing or treating sepsis and sepsis-induced acute kidney injury according to claim 6, wherein the galactolipids-enriched fraction obtained in step (c) is further purified by a reversed phase high performance liquid chromatography to have a purified compound, wherein the galactolipids-enriched fraction is eluted by a alcohol elution buffer and the alcohol is 70 % to 100% volume percentage of the alcohol elution buffer, wherein the purified compound is 1,2,di-O-α-linolenoyl-3-O-β-galactopyranosyl-sn-glycerol (dLGG).

8. The composition for use according to claim 6, wherein the alcohol elution buffer for use in step (c) comprises methanol and water, methanol and dichloromethane, methanol and acetonitrile or methanol and acetone.

9. The composition for use according to claim 8, wherein the alcohol elution buffer is used to elute an ethyl acetate extract from *Gynura divaricata subsp. formosana.*

10. The composition for use according to claim 8, wherein the alcohol elution buffer is used to elute an ethyl acetate extract from *Murdannia bracteata (C. B. Clarke) J. K. Morton ex D. Y. Hong.*

11. The composition for use according to claim 6, wherein the related symptom is an acute kidney injury induced by sepsis.

**12.** A non-medical use for skin whitening of a composition, comprising:

a pharmaceutical, healthy or food acceptable vehicle, and
an effective amount of a bioactive ingredient, wherein the bioactive ingredient is a galactolipids-enriched plant extract or a purified compound obtained from purification of the galactolipids-enriched plant extract, wherein the galactolipids-enriched plant extract is obtained by the steps of:

(a) using water, methanol or ethanol to extract a plant sample, wherein the plant sample is selected from a group consisting of: *Gynura divaricata subsp. formosana, Murdannia bracteata* (*C. B. Clarke*) *J. K. Morton ex D. Y. Hong,* and *Crassocephalum rabens S. Moore;*
(b) using ethyl acetate to partition the extract from step (a) to obtain an ethyl acetate extract;
(c) using an alcohol elution buffer for eluting a galactolipids-enriched fraction from the ethyl acetate extract from step (b), wherein the alcohol is 5% to 20 % volume percentage of the alcohol elution buffer, and

wherein the galactolipids-enriched fraction is a 1,2,di-O-$\alpha$-linolenoyl-3-O-$\beta$-galactopyranosyl-sn-glycerol (dLGG)-enriched fraction.

**13.** The non-medical use for skin whitening according to claim 12, wherein the galactolipids-enriched fraction obtained in step (c) is further purified by a reversed phase high performance liquid chromatography to have a purified galactolipid-containing compound, wherein the galactolipids-enriched fraction is eluted by a alcohol elution buffer and the alcohol is 70 % to 100% volume percentage of the alcohol elution buffer, wherein the purified compound is 1,2,di-O-$\alpha$-linolenoyl-3-O-$\beta$-galactopyranosyl-sn-glycerol (dLGG).

**14.** The non-medical use for skin whitening according to claim 12, wherein the alcohol elution buffer for use in step (c) comprises: methanol and water, methanol and dichloromethane, or methanol and acetonitrile.

**15.** The non-medical use for skin whitening according to claim 14, wherein the alcohol elution buffer is used to elute an ethyl acetate extract from *Gynura divaricata subsp. formosana.*

**16.** The non-medical use for skin whitening according to claim 14, wherein the alcohol elution buffer is used to elute an ethyl acetate extract from *Murdannia bracteata* (C. *B. Clarke) J. K. Morton ex D. Y. Hong.*

**Patentansprüche**

**1.** Zusammensetzung eines Arzneimittel-, gesundheitsfördernden oder Nahrungszusatzstoffes für die Anwendung zur Vorbeugung oder Behandlung von akuter fulminanter Hepatitis durch Senken der Serumspiegel von Aspartat-Aminotransferase (AST) und Alanin-Aminotransferase (ALT), welche umfasst:

eine pharmazeutische, gesundheitsfördernde oder lebensmittelverträgliche Trägersubstanz, und
eine effektive Menge eines bioaktiven Inhaltsstoffs, wobei der bioaktive Inhaltsstoff ein mit Galaktolipiden angereichertes Pflanzenextrakt oder eine gereinigte Verbindung ist, welche durch Aufreinigung des mit Galaktolipiden angereicherten Pflanzenextrakts erhalten wird, wobei das mit Galaktolipiden angereicherte Pflanzenextrakt durch die folgenden Schritte erhalten wird:

(a) Verwendung von Wasser, Methanol oder Ethanol zum Extrahieren einer Pflanzenprobe, wobei die Pflanzenprobe ausgewählt ist aus einer Gruppe, die *Gynura divaricata subsp. formosana, Murdannia bracteata (C. B. Clarke) J. K. Morton ex D. Y. Hong* und *Crassocephalum rabens S. Moore* umfasst;
(b) Verwendung von Ethylacetat zum Teilen des Extrakts aus Schritt (a), um ein Ethylacetat-Extrakt zu erhalten;
(c) Verwendung eines Alkoholelutionspuffers zum Eluieren einer mit Galaktolipiden angereicherten Fraktion aus dem Ethylacetat-Extrakt aus Schritt (b), wobei der Alkohol 5% bis 20 % in Volumenprozent des Alkoholelutionspuffers ausmacht,

wobei die mit Galaktolipiden angereicherte Fraktion eine Fraktion ist, in der 1,2-Di-O-$\alpha$-linolenoyl-3-O-$\beta$-galactopyranosyl-sn-glycerol (dLGG) angereichert ist.

**2.** Zusammensetzung für die Anwendung zur Vorbeugung oder Behandlung von akuter fulminanter Hepatitis nach Anspruch 1, bei welcher die im Schritt (c) erhaltene mit Galaktolipiden angereicherte Fraktion ferner durch eine Umkehrphasenhochleistungsflüssigkeitschromatographie gereinigt wird, um eine gereinigte Verbindung zu erhalten, wobei die mit Galaktolipiden angereicherte Fraktion durch einen Alkoholelutionspuffer eluiert wird und der Alkohol 70 % bis 100 % in Volumenprozent des Alkoholelutionspuffers ausmacht, wobei die gereinigte Verbindung 1,2-Di-O-$\alpha$-linolenoyl-3-O-$\beta$-galactopyranosyl-sn-glycerol (dLGG) ist.

3. Zusammensetzung für die Anwendung zur Vorbeugung oder Behandlung von akuter fulminanter Hepatitis nach Anspruch 1, bei welcher der Alkoholelutionspuffer zur Verwendung im Schritt (c) Methanol und Wasser, Methanol und Dichlormethan, Methanol und Acetonitril oder Methanol und Aceton umfasst.

4. Zusammensetzung für die Anwendung zur Vorbeugung oder Behandlung von akuter fulminanter Hepatitis nach Anspruch 3, bei welcher der Alkoholelutionspuffer verwendet wird, um ein Ethylacetat-Extrakt von *Gynura divaricata subsp. formosana* zu eluieren.

5. Zusammensetzung für die Anwendung zur Vorbeugung oder Behandlung von akuter fulminanter Hepatitis nach Anspruch 3, bei welcher der Alkoholelutionspuffer verwendet wird, um ein Ethylacetat-Extrakt von *Murdannia bracteata (C. B. Clarke) J. K. Morton ex D. Y. Hong zu* eluieren.

6. Zusammensetzung eines Arzneimittel-, gesundheitsfördernden oder Nahrungszusatzstoffes für die Anwendung zur Vorbeugung oder Behandlung von Symptomen einer Sepsis und sepsis-induzierten akuten Nierenschädigung, welche mit einer verstärkten Expression des Hypoxie-induzierbaren Faktors-1$\alpha$ (HIF-1$\alpha$) assoziiert sind, welche umfasst:

eine pharmazeutische, gesundheitsfördernde oder lebensmittelverträgliche Trägersubstanz, und eine effektive Menge eines bioaktiven Inhaltsstoffs, wobei der bioaktive Inhaltsstoff ein mit Galaktolipiden angereichertes Pflanzenextrakt oder eine gereinigte Verbindung ist, welche durch Aufreinigung des mit Galaktolipiden angereicherten Pflanzenextrakts erhalten wird, wobei das mit Galaktolipiden angereicherte Pflanzenextrakt durch die folgenden Schritte erhalten wird:

(a) Verwendung von Wasser, Methanol oder Ethanol zum Extrahieren einer Pflanzenprobe, wobei die Pflanzenprobe ausgewählt ist aus einer Gruppe, die *Gynura divaricata subsp. formosana, Murdannia bracteata (C. B. Clarke) J. K. Morton ex D. Y. Hong* und *Crassocephalum rabens S. Moore* umfasst;
(b) Verwendung von Ethylacetat zum Teilen des Extrakts aus Schritt (a), um ein Ethylacetat-Extrakt zu erhalten;
(c) Verwendung eines Alkoholelutionspuffers zum Eluieren einer mit Galaktolipiden angereicherten Fraktion aus dem Ethylacetat-Extrakt aus Schritt (b), wobei der Alkohol 5 % bis 20 % in Volumenprozent des Alkoholelutionspuffers ausmacht, und

wobei die mit Galaktolipiden angereicherte Fraktion eine Fraktion ist, in der 1,2-Di-O-$\alpha$-linolenoyl-3-O-$\beta$-galactopyranosyl-sn-glycerol (dLGG) angereichert ist.

7. Zusammensetzung für die Anwendung zur Vorbeugung oder Behandlung einer Sepsis und sepsis-induzierten akuten Nierenschädigung nach Anspruch 6, bei welcher die im Schritt (c) erhaltene mit Galaktolipiden angereicherte Fraktion ferner durch eine Umkehrphasenhochleistungsflüssigkeitschromatographie gereinigt wird, um eine gereinigte Verbindung zu erhalten, wobei die mit Galaktolipiden angereicherte Fraktion durch einen Alkoholelutionspuffer eluiert wird und der Alkohol 70 % bis 100 % in Volumenprozent des Alkoholelutionspuffers ausmacht, wobei die gereinigte Verbindung 1,2-Di-O-$\alpha$-linolenoyl-3-O-$\beta$-galactopyranosyl-sn-glycerol (dLGG) ist.

8. Zusammensetzung für die Anwendung nach Anspruch 6, bei welcher der Alkoholelutionspuffer zur Verwendung im Schritt (c) Methanol und Wasser, Methanol und Dichlormethan, Methanol und Acetonitril oder Methanol und Aceton umfasst.

9. Zusammensetzung für die Anwendung nach Anspruch 8, bei welcher der Alkoholelutionspuffer verwendet wird, um ein Ethylacetat-Extrakt von *Gynura divaricata subsp. formosana* zu eluieren.

10. Zusammensetzung für die Anwendung nach Anspruch 8, bei welcher der Alkoholelutionspuffer verwendet wird, um ein Ethylacetat-Extrakt von *Murdannia bracteata (C. B. Clarke) J. K. Morton ex D. Y. Hong* zu eluieren.

11. Zusammensetzung für die Anwendung nach Anspruch 6, bei welcher das zugehörige Symptom ein durch eine Sepsis induzierter akuter Nierenschaden ist.

12. Nicht-medizinische Anwendung zur Hautaufhellung einer Zusammensetzung, welche umfasst:

eine pharmazeutische, gesundheitsfördernde oder lebensmittelverträgliche Trägersubstanz, und
eine effektive Menge eines bioaktiven Inhaltsstoffs, wobei der bioaktive Inhaltsstoff ein mit Galaktolipiden angereichertes Pflanzenextrakt oder eine gereinigte Verbindung ist, welche durch Aufreinigung des mit Galaktolipiden angereicherten Pflanzenextrakts erhalten wird, wobei das mit Galaktolipiden angereicherte Pflanzenextrakt durch die folgenden Schritte erhalten wird:

(a) Verwendung von Wasser, Methanol oder Ethanol zum Extrahieren einer Pflanzenprobe, wobei die Pflanzenprobe ausgewählt ist aus einer Gruppe, die *Gynura divaricata subsp. formosana, Murdannia bracteata (C. B. Clarke) J. K. Morton ex D. Y. Hong* und *Crassocephalum rabens S. Moore* umfasst;
(b) Verwendung von Ethylacetat zum Teilen des Extrakts aus Schritt (a), um ein Ethylacetat-Extrakt zu erhalten;
(c) Verwendung eines Alkoholelutionspuffers zum Eluieren einer mit Galaktolipiden angereicherten Fraktion aus dem Ethylacetat-Extrakt aus Schritt (b), wobei der Alkohol 5 % bis 20 % in Volumenprozent des Alkoholelutionspuffers ausmacht, und

wobei die mit Galaktolipiden angereicherte Fraktion eine Fraktion ist, in der 1,2-Di-O-$\alpha$-linolenoyl-3-O-$\beta$-galactopyranosyl-sn-glycerol (dLGG) angereichert ist.

13. Nicht-medizinische Anwendung zur Hautaufhellung nach Anspruch 12, bei welcher die im Schritt (c) erhaltene mit Galaktolipiden angereicherte Fraktion ferner durch eine Umkehrphasenhochleistungsflüssigkeitschromatographie gereinigt wird, um eine gereinigte galaktolipidhaltige Verbindung zu erhalten, wobei die mit Galaktolipiden angereicherte Fraktion durch einen Alkoholelutionspuffer eluiert wird und der Alkohol 70 % bis 100 % in Volumenprozent des Alkoholelutionspuffers ausmacht, wobei die gereinigte Verbindung 1,2-Di-O-$\alpha$-linolenoyl-3-O-$\beta$-galactopyranosyl-sn-glycerol (dLGG) ist.

14. Nicht-medizinische Anwendung zur Hautaufhellung nach Anspruch 12, bei welcher der Alkoholelutionspuffer zur Verwendung im Schritt (c) Methanol und Wasser, Methanol und Dichlormethan oder Methanol und Acetonitril umfasst.

15. Nicht-medizinische Anwendung zur Hautaufhellung nach Anspruch 14, bei welcher der Alkoholelutionspuffer verwendet wird, um ein Ethylacetat-Extrakt von *Gynura divaricata subsp. formosana* zu eluieren.

16. Nicht-medizinische Anwendung zur Hautaufhellung nach Anspruch 14, bei welcher der Alkoholelutionspuffer verwendet wird, um ein Ethylacetat-Extrakt von *Murdannia bracteata (C. B. Clarke) J. K. Morton ex D. Y. Hong* zu eluieren.


**Revendications**

1. Composition d'additif pharmaceutique, bon pour la santé ou nutritionnel pour une utilisation dans la prévention ou le traitement d'une hépatite fulminante aiguë en diminuant les taux sériques d'aspartate aminotransférase (AST) et d'alanine aminotransférase (ALT), comprenant :

un véhicule pharmaceutique, bon pour la santé ou de qualité alimentaire, et
une quantité efficace d'un ingrédient bioactif, dans laquelle l'ingrédient bioactif est un extrait végétal enrichi en galactolipides ou un composé purifié obtenu à partir de la purification de l'extrait végétal enrichi en galactolipides, dans laquelle l'extrait végétal enrichi en galactolipides est obtenu par les étapes consistant à :

(a) utiliser de l'eau, du méthanol ou de l'éthanol pour extraire un échantillon végétal, dans laquelle l'échantillon végétal est choisi dans un groupe constitué de : *Gynura divaricata sous-espèce formosana, Murdannia bracteata (C. B. Clarke) J. K. Morton ex D. Y. Hong,* et *Crassocephalum rabens S. Moore* ;
(b) utiliser de l'acétate d'éthyle pour séparer l'extrait provenant de l'étape (a) pour obtenir un extrait à l'acétate d'éthyle ;

(c) utiliser un tampon d'élution alcoolique pour éluer une fraction enrichie en galactolipides à partir de l'extrait à l'acétate d'éthyle provenant de l'étape (b), dans laquelle l'alcool représente un pourcentage en volume de 5 % à 20 % du tampon d'élution alcoolique,

dans laquelle la fraction enrichie en galactolipides est une fraction enrichie en 1,2,di-O-α-linolénoyl-3-O-β-galactopyranosyl-sn-glycérol (dLGG).

2. Composition pour utilisation dans la prévention ou le traitement d'une hépatite fulminante aiguë selon la revendication 1, dans laquelle la fraction enrichie en galactolipides obtenue à l'étape (c) est encore purifiée par une chromatographie liquide à haute performance à inversion de phase pour avoir un composé purifié, dans laquelle la fraction enrichie en galactolipides est éluée par un tampon d'élution alcoolique et l'alcool représente un pourcentage en volume de 70 % à 100 % du tampon d'élution alcoolique, dans laquelle le composé purifié est du 1,2,di-O-α-linolénoyl-3-O-β-galactopyranosyl-sn-glycérol (dLGG).

3. Composition pour utilisation dans la prévention ou le traitement d'une hépatite fulminante aiguë selon la revendication 1, dans laquelle le tampon d'élution alcoolique pour utilisation à l'étape (c) comprend du méthanol et de l'eau, du méthanol et du dichlorométhane, du méthanol et de l'acétonitrile ou du méthanol et de l'acétone.

4. Composition pour utilisation dans la prévention ou le traitement d'une hépatite fulminante aiguë selon la revendication 3, dans laquelle le tampon d'élution alcoolique est utilisé pour éluer un extrait à l'acétate d'éthyle de *Gynura divaricata sous-espèce formosana.*

5. Composition pour utilisation dans la prévention ou le traitement d'une hépatite fulminante aiguë selon la revendication 3, dans laquelle le tampon d'élution alcoolique est utilisé pour éluer un extrait à l'acétate d'éthyle de *Murdannia bracteata (C. B. Clarke) J. K. Morton ex D. Y. Hong.*

6. Composition d'additif pharmaceutique, bon pour la santé ou nutritionnel pour une utilisation dans la prévention ou le traitement de symptômes de septicémie et d'insuffisance rénale aiguë induite par septicémie, associés à une expression accrue de facteur inductible par l'hypoxie-1α (HIF-1α), comprenant :

un véhicule pharmaceutique, bon pour la santé ou de qualité alimentaire, et
une quantité efficace d'un ingrédient bioactif, dans laquelle l'ingrédient bioactif est un extrait végétal enrichi en galactolipides ou un composé purifié obtenu à partir de la purification de l'extrait végétal enrichi en galactolipides, dans laquelle l'extrait végétal enrichi en galactolipides est obtenu par les étapes consistant à :

(a) utiliser de l'eau, du méthanol ou de l'éthanol pour extraire un échantillon végétal, dans laquelle l'échantillon végétal est choisi dans un groupe constitué de : *Gynura divaricata sous-espèce formosana, Murdannia bracteata (C. B. Clarke) J. K. Morton ex D. Y. Hong,* et *Crassocephalum rabens S. Moore* ;
(b) utiliser de l'acétate d'éthyle pour séparer l'extrait provenant de l'étape (a) pour obtenir un extrait à l'acétate d'éthyle ;
(c) utiliser un tampon d'élution alcoolique pour éluer une fraction enrichie en galactolipides à partir de l'extrait à l'acétate d'éthyle provenant de l'étape (b), dans laquelle l'alcool représente un pourcentage en volume de 5 % à 20 % du tampon d'élution alcoolique, et

dans laquelle la fraction enrichie en galactolipides est une fraction enrichie en 1,2,di-O-α-linolénoyl-3-O-β-galactopyranosyl-sn-glycérol (dLGG).

7. Composition pour utilisation dans la prévention ou le traitement d'une septicémie et d'une insuffisance rénale aiguë induite par septicémie selon la revendication 6, dans laquelle la fraction enrichie en galactolipides obtenue à l'étape (c) est encore purifiée par une chromatographie liquide à haute performance à inversion de phase pour avoir un composé purifié, dans laquelle la fraction enrichie en galactolipides est éluée par un tampon d'élution alcoolique et l'alcool représente un pourcentage en volume de 70 % à 100 % du tampon d'élution alcoolique, dans laquelle le composé purifié est du 1,2,di-O-α-linolénoyl-3-O-β-galactopyranosyl-sn-glycérol (dLGG).

8. Composition pour utilisation selon la revendication 6, dans laquelle le tampon d'élution alcoolique pour utilisation à l'étape (c) comprend du méthanol et de l'eau, du méthanol et du dichlorométhane, du méthanol et de l'acétonitrile ou du méthanol et de l'acétone.

**9.** Composition pour utilisation selon la revendication 8, dans laquelle le tampon d'élution alcoolique est utilisé pour éluer un extrait à l'acétate d'éthyle de *Gynura divaricata sous-espèce formosana.*

**10.** Composition pour utilisation selon la revendication 8, dans laquelle le tampon d'élution alcoolique est utilisé pour éluer un extrait à l'acétate d'éthyle de *Murdannia bracteata (C. B. Clarke) J. K. Morton ex D. Y. Hong.*

**11.** Composition pour utilisation selon la revendication 6, dans laquelle le symptôme apparenté est une insuffisance rénale aiguë induite par une septicémie.

**12.** Utilisation non médicale pour un blanchiment de la peau d'une composition, comprenant :

un véhicule pharmaceutique, bon pour la santé ou de qualité alimentaire, et
une quantité efficace d'un ingrédient bioactif, dans laquelle l'ingrédient bioactif est un extrait végétal enrichi en galactolipides ou un composé purifié obtenu à partir de la purification de l'extrait végétal enrichi en galactolipides, dans laquelle l'extrait végétal enrichi en galactolipides est obtenu par les étapes consistant à :

(a) utiliser de l'eau, du méthanol ou de l'éthanol pour extraire un échantillon végétal, dans laquelle l'échantillon végétal est choisi dans un groupe constitué de : *Gynura divaricata sous-espèce formosana, Murdannia bracteata (C. B. Clarke) J. K. Morton ex D. Y. Hong,* et *Crassocephalum rabens S. Moore* ;
(b) utiliser de l'acétate d'éthyle pour séparer l'extrait provenant de l'étape (a) pour obtenir un extrait à l'acétate d'éthyle ;
(c) utiliser un tampon d'élution alcoolique pour éluer une fraction enrichie en galactolipides à partir de l'extrait à l'acétate d'éthyle provenant de l'étape (b), dans laquelle l'alcool représente un pourcentage en volume de 5 % à 20 % du tampon d'élution alcoolique, et

dans laquelle la fraction enrichie en galactolipides est une fraction enrichie en 1,2,di-O-$\alpha$-linolénoyl-3-O-$\beta$-galactopyranosyl-sn-glycérol (dLGG).

**13.** Utilisation non médicale pour un blanchiment de la peau selon la revendication 12, dans laquelle la fraction enrichie en galactolipides obtenue à l'étape (c) est en outre purifiée par une chromatographie liquide à haute performance à inversion de phase pour avoir un composé purifié contenant des galactolipides, dans laquelle la fraction enrichie en galactolipides est éluée par un tampon d'élution alcoolique et l'alcool représente un pourcentage en volume de 70 % à 100 % du tampon d'élution alcoolique, dans laquelle le composé purifié est du 1,2,di-O-$\alpha$-linolénoyl-3-O-$\beta$-galactopyranosyl-sn-glycérol (dLGG).

**14.** Utilisation non médicale pour un blanchiment de la peau selon la revendication 12, dans laquelle le tampon d'élution alcoolique pour utilisation à l'étape (c) comprend : du méthanol et de l'eau, du méthanol et du dichlorométhane, ou du méthanol et de l'acétonitrile.

**15.** Utilisation non médicale pour un blanchiment de la peau selon la revendication 14, dans laquelle le tampon d'élution alcoolique est utilisé pour éluer un extrait à l'acétate d'éthyle de *Gynura divaricata sous-espèce formosana.*

**16.** Utilisation non médicale pour un blanchiment de la peau selon la revendication 14, dans laquelle le tampon d'élution alcoolique est utilisé pour éluer un extrait à l'acétate d'éthyle de *Murdannia bracteata (C. B. Clarke) J. K. Morton ex D. Y. Hong.*

Fig. 1A

$R_1/R_2$:   (1)

$R_1/R_2$:   or   (2)

$R_1/R_2$:   (3)

$R_1/R_2$:   or   (4)

$R_1/R_2$:   or   (5)

Fig. 1B

EP 2 810 651 B1

Fig. 2A

Fig. 2B

EP 2 810 651 B1

Fig. 3

Fig. 4

Fig. 5

Fig. 6

Fig. 7

Fig. 8

Fig. 9A

Fig. 9B

Fig. 9C

Fig. 9D

Fig. 10

**EP 2 810 651 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008105436 A1
- WO 2012021068 A1
- CN 101152340 A
- US 2008152737 A1

### Non-patent literature cited in the description

- **PHAN VAN KIEM et al.** *Archives of Pharmacal Research,* vol. 35, 2135-2142
- **MUN FEI YAM et al.** *Journal of Acupuncture and Meridian Studies,* vol. 3, 197-202
- **NAKAMURA SEIKOU et al.** *Chemical and Pharmaceutical Bulletin,* vol. 59, 1020-1028
- **HARRY MARTIN et al.** *Bioscience Reports,* vol. 230, 151f
- **C.-C. HOU et al.** *Cancer Research,* vol. 67, 6907-6915
- **HOU.** *Cancer Research,* 2007, vol. 67, 6907-6915